# EUROPEAN PATENT APPLICATION

(11) **EP 1 600 776 A2**
(43) Date of publication of application: **30.11.2005**
(21) Application number: 05253215.7
(22) Date of filing: 25.05.2005
(51) Int. Cl.: G01N 33/68

(54) **Peptide mass spectrometry**

(30) Priority: 25.05.2004 GB 0411687
(71) Applicant: OXFORD GENE TECHNOLOGY IP LIMITED, Sandy Lane, Yarnton, Oxford OX5 1PF (GB)
(72) Inventor: Wheeler, Susan, Oxford Gene Technology IP Ltd, Yarnton, Oxford OX5 1PF (GB); Shchepinov, Mikhail S., Oxford Gene Techn. IP Ltd, Yarnton, Oxford OX5 1PF (GB); Southern, Edwin M., Oxford Gene Technology IP Ltd, Yarnton, Oxford OX5 1PF (GB)
(74) Representative: Marshall, Cameron John

(57) **Abstract**

Methods of analysing peptides by mass spectrometry are disclosed. In particular, methods of analysing peptides by fragmentation mass spectrometry (MSⁿ, where n is at least 2) are disclosed. The methods involve derivatisation of a peptide at its N-terminus such that peaks corresponding to both a and b (and y) daughter ions are identifiable in fragmentation mass spectra of the derivatised peptide. The fragmentation mass spectra of the derivatised peptide contain additional information (relative to the fragmentation mass spectra of the underivatised peptide) useful for determination of the amino acid sequence of the peptide.

### Annex to the application documents - subsequently filed sequences listing

SEQUENCE LISTING

<110> OXFORD GENE TECHNOLOGY IP LIMITED

<120> PEPTIDE MASS SPECTROMETRY RICH IN DAUGHTER IONS

<130> P037830EP

<140> EP 05253215.7
<141> 2005-05-25

<150> GB 0411687.7
<151> 2004-05-25

<160> 5

<170> SeqWin99, version 1.02

<210> 1
<211> 12
<212> PRT
<213> Artificial Sequence

<220>
<223> Derivatised peptide

<400> 1
Arg His Pro Glu Tyr Ala Val Ser Val Leu Leu Arg
1               5                   10

<210> 2
<211> 13
<212> PRT
<213> Artificial Sequence

<220>
<223> Derivatised peptide

<400> 2
Asp Asp Pro His Ala Cys Tyr Ser Thr Val Phe Asp Lys
1               5                   10

<210> 3
<211> 8
<212> PRT
<213> Artificial Sequence

<220>
<223> Derivatised peptide

<400> 3
Glu Ala Leu Asp Phe Phe Ala Arg
1               5

<210> 4
<211> 7
<212> PRT
<213> Artificial Sequence

<220>
<223> Derivatised peptide

<400>   4
Gly Pro Phe Pro Ile Ile Val
1               5

<210>   5
<211>   14
<212>   PRT
<213>   Artificial Sequence

<220>
<223>   GluFib B peptide

<400>   5

## Description

### TECHNICAL FIELD

This invention relates to methods of analysing peptides by mass spectrometry. In particular, the invention relates to methods of analysing peptides by fragmentation mass spectrometry (MSⁿ, where n is at least 2).

### BACKGROUND OF THE INVENTION

Mass spectrometry is a powerful method for identification of proteins or peptides in a sample. Typically, "peptide mass fingerprinting" is used to identify a protein of interest via database searching. The potency of this method continues to grow due to improvements to the search algorithms and rapid expansion of the relevant databases. However, it is not always possible to unambiguously identify a protein of interest by peptide mass fingerprinting.

Firstly, there are a number of factors that can reduce the efficiency of peptide mass fingerprinting, and as a result a large number of false positive matches are often returned by the database search. Secondly, peptide mass fingerprinting can only be used to identify proteins which are present in the relevant databases (or proteins that have a high level of sequence identity to such proteins) and so cannot be used for the identification of unknown proteins. However, fragmentation mass spectrometry (also known as "tandem mass spectrometry") permits a *de novo* investigation of amino acid sequence, and allows these problems to be reduced or overcome.

For example, false positive matches may be reduced or avoided by incorporating amino acid sequence information into a peptide mass fingerprinting database search. There is no need to determine the entire amino acid sequence of the protein of interest, since a consecutive series of three or four amino acids can provide enough search specificity, when combined with the peptide mass fingerprint, to enable unambiguous identification of the protein of interest. Secondly, the amino acid sequence information may be sufficient to enable cloning of the gene (for example, by enabling design of degenerate PCR primers) or may be sufficient to allow full-length *de novo* sequencing of the protein of interest. Accordingly, fragmentation mass spectrometry has become a very important method in proteomics research.

Fragmentation mass spectrometry involves the selection of one or more parent ions from the peptide ions analysed in a first detection step, fragmentation of the selected parent ion(s) to provide a number of daughter ions, and analysis of the daughter ions in a second detection step. The mass spectrum of the mixture of daughter ions provides information regarding the amino acid sequence of the parent ion. Further iterations may be performed, which each involve an additional cycle of parent ion selection, fragmentation to produce daughter ions and daughter ion detection. Thus, fragmentation mass spectrometry is also referred to as MSⁿ, where n is the number of iterations of peptide ion detection (MS² is often referred to as MS/MS or tandem mass spectrometry).

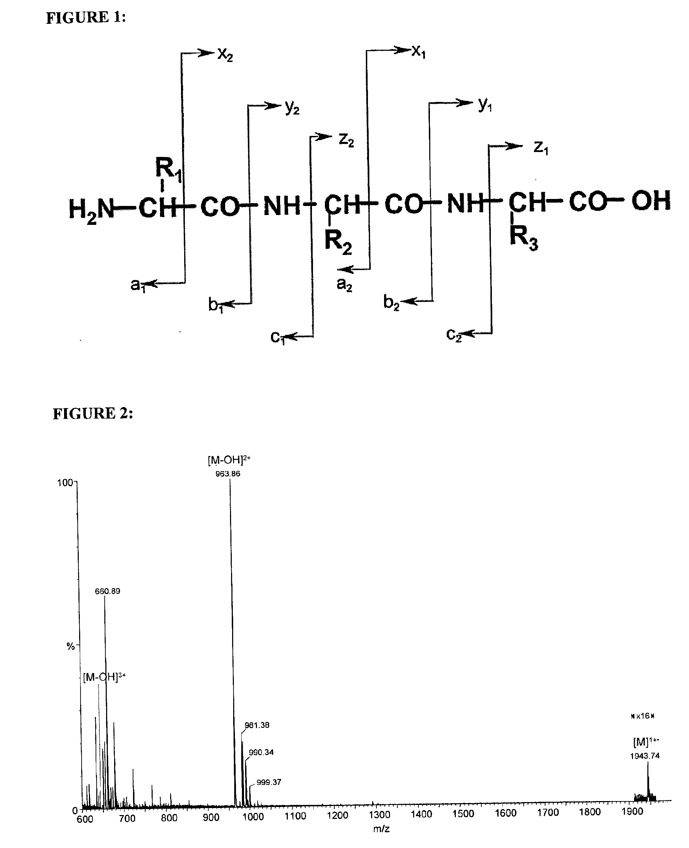

The major daughter ion types generated by cleavage of the peptide backbone are known as a, b, c and x, y, z ions (see Figure 1). The accepted nomenclature for daughter ions was been developed by Biemann (see Biemann K., Nomenclature for Peptide Fragment Ions (Positive ions), in Methods in Enzymology: Mass Spectrometry, ed. J. A. McCloskey, Academic Press, Inc. San Diego, CA, 1990, 886-887). Cleavage of the CO-NH (peptide) bond generates N-terminal b- and C-terminal y-daughter ions. Cleavage of the CH(R)-CO bond generates N-terminal a- and C-terminal x- daughter ions. Cleavage of the NH-CH(R) bond generates N-terminal c- and C-terminal z- daughter ions.

Cleavage of the equivalent bond at each location along the peptide backbone provides a series of daughter ions. By comparing the successive mass-to-charge ratios (m/z) of the ions within a series (for example, the y ion series) with the known masses of amino acid residues, the amino acid sequence of the parent ion can be determined. Ideally, the sequence of a peptide could be determined by simple conversion of the "ladder" of m/z values for the consecutive daughter ions in each series to the corresponding amino acid sequence. Fragmentation mass spectrometry theoretically permits *de novo* sequencing of entire proteins, by re-assembly of the parent ion sequences.

However, a simple ladder spectrum will only be produced by cleavage of the equivalent bond at each location along the peptide backbone, and in practice the fragmentation process is far from ideal. As the parent ions become larger, a smaller proportion of the backbone bonds are cleaved, and thus complete ion series may not be obtained. In addition, the presence of a proline residue may prevent complete fragmentation of the peptide. As a result, the fragmentation mass spectrum may not contain the information necessary for the complete amino acid sequence of the parent ion to be determined.

*De novo* sequencing of peptides by fragmentation mass spectrometry remains technically challenging and requires extensive input from expert mass spectrometrists, despite a number of attempts having been made to overcome the problems described above.

For example, attempts have been made to increase the level of peptide backbone fragmentation by using new MS instrumentation. For example, "collision-induced dissociation" (CID; also referred to as CAD or collisional-activated dissociation) or "post-source decay" (PSD) may increase the number of daughter ions available for analysis.

For example, chemical derivatisation has been used to influence the generation of particular daughter ion types. Keough *et al*. (Rapid Commun Mass Spectrom. 2000;14(24):2348-56 and WO 02/08767) introduce a negatively charged group at the N-terminus of the peptides. As a result, any N-terminal daughter ions are neutral (and undetected), whilst C-terminal fragments retain a positive charge (and are detected). This derivatisation is reported to result in MSⁿ spectra that are free of a and b daughter ions. In addition, this derivatisation is reported to increase the level of PSD (this effect is referred to by Keough *et al.* as "chemically-assisted fragmentation"), such that a full y ion series is detected.

There remains a need for improvements in *de novo* sequencing of proteins and peptides by fragmentation mass spectrometry.

### DISCLOSURE OF THE INVENTION

The inventors have found that a peptide can be derivatised at its N-terminus such that peaks corresponding to both a and b daughter ions are identifiable in fragmentation mass spectra of the derivatised peptide. As a result, the fragmentation mass spectra of the derivatised peptide contain additional information (relative to the fragmentation mass spectra of the underivatised peptide) useful for determination of the amino acid sequence of the peptide.

The invention therefore provides a method for analysing a peptide by mass spectrometry, comprising the step of obtaining a fragmentation mass spectrum for the peptide, wherein the fragmentation mass spectrum contains peaks corresponding to both a and b daughter ions.

In particular, the spectrum contains peaks corresponding to a and b daughter ions formed by cleavage between the same two amino acids but at different bonds.

The step of obtaining a fragmentation mass spectrum may comprise the steps of:
(a) reacting the peptide's N-terminus with a label to provide a derivatised peptide, wherein the label is an organic group that allows positive charge to be retained by a and b ions formed during fragmentation mass spectrometry of the derivatised peptide; and
(b) providing a fragmentation mass spectrum of the derivatised peptide, e.g. by fragmentation mass spectrometry (MSⁿ, wherein n is at least 2).

For instance, this step (b) may comprise the steps of:
(b1) performing mass spectrometry of the derivatised peptide to obtain a first mass spectrum;
(b2) identifying at least one parent ion in the first mass spectrum;
(b3) selecting at least one parent ion for further analysis by mass spectrometry; and
(b4) performing fragmentation mass spectrometry of the selected parent ion(s) to obtain a fragmentation mass spectrum, wherein the fragmentation mass spectrum contains peaks corresponding to both a and b ions derived from fragmentation of the derivatised peptide.

The presence of peaks corresponding to both a and b ions in the fragmentation mass spectra generated in the methods of the present invention facilitates the assignment of amino acid sequence information to the peptide. The presence of both a and b ions in the fragmentation mass spectra provides two separate routes for *de novo* sequence assignment from the N-terminus to the C-terminus of the peptide.

Thus, the invention provides a method for determining an amino acid sequence of a peptide, comprising the steps of:
(a) obtaining a fragmentation mass spectrum for the peptide, wherein the fragmentation mass spectrum contains peaks corresponding to both a and b daughter ions;
(b) identifying peaks in the fragmentation mass spectrum of step (a) corresponding to a or b daughter ions, preferably identifying peaks corresponding to both a and b daughter ions; and
(c) assigning an amino acid sequence to the peptide on the basis of the m/z separation of consecutive peaks in the a or b ion series.

As illustrated by Figure 1, the mass difference between corresponding a and b ions is the mass of a carbonyl group (28 Da). The fragmentation mass spectra generated in the methods of the present invention comprise a number of aₙ and bₙ ion peak pairs, wherein a peak corresponding to an aₙ ion is separated from a peak corresponding to a bₙ ion by the m/z of a carbonyl group (28 Da/z). These characteristic pairs of a and b ion peaks are referred to herein as a/b doublets.

The invention thus provides a method for analysing a peptide by mass spectrometry, comprising the step of obtaining a fragmentation mass spectrum for the peptide, wherein the fragmentation mass spectrum contains at least one a/b doublet. Preferably, the fragmentation mass spectrum contains a series of a/b doublets (for example, at least 30%, 40%, 50%, 60%, 70%, 80%, 90% or even 100% of the theoretical a/b doublets of a given peptide), and the m/z separation between consecutive a and b ions of consecutive a/b doublets corresponds to that of an amino acid, and thus enables an amino acid sequence to be assigned to the peptide. Preferably, the fragmentation mass spectrum contains a/b doublets for at least 30% (e.g. 40%, 50%, 60%, 70%, 80%, 90% or even 100%) of the peptide's amino acid residues.

In the known methods of fragmentation mass spectrometry it not usual to observe two daughter ion peaks separated by a m/z of 28 Da/z. This peak pattern would normally only be observed following an unusual side-chain cleavage event. Thus, the presence of a series a/b doublets in the fragmentation mass spectra generated in the methods of the present invention enables rapid assignment of a and b ion types to the peaks in the fragmentation mass spectra (the a ion of each a/b doublet will have the lower m/z). In turn, the m/z separation between adjacent pairs of a/b doublets corresponds to that of an amino acid, and thus enables an amino acid sequence to be assigned to the peptide.

The inventors have also found that a peptide can be derivatised at its N-terminus such that peaks corresponding to each of a, b and y ions are identifiable in fragmentation mass spectra of the derivatised peptide. As a result, the fragmentation mass spectra of the derivatised peptide contain further additional information (relative to the fragmentation mass spectra of the underivatised peptide) useful for determination of the amino acid sequence of the peptide.

Thus, the invention also provides a method for analysing a peptide by mass spectrometry, comprising the step of obtaining a fragmentation mass spectrum for the peptide, wherein the fragmentation mass spectrum contains peaks corresponding to each of a, b and y daughter ions. The peptide may be derivatised as described above.

The step of obtaining a fragmentation mass spectrum for the peptide may comprise the steps of:
(b1) performing mass spectrometry of the derivatised peptide to obtain a first mass spectrum;
(b2) identifying at least one parent ion in the first mass spectrum;
(b3) selecting at least one parent ion for further analysis by mass spectrometry; and
(b4) performing fragmentation mass spectrometry of the selected parent ion(s) to obtain a fragmentation mass spectrum, wherein the fragmentation mass spectrum contains peaks corresponding to each of a, b and y ions derived from fragmentation of the peptide.

The presence of additional peaks corresponding to y ions in the mass spectrum of daughter ions derived from fragmentation of derivatised peptides further facilitates the assignment of amino acid sequence information to the parent ion. The presence of each of a, b and y ions in the fragmentation mass spectra provides three separate routes for *de novo* sequence assignment. The presence of y ions allows *de novo* sequence assignment from the C-terminus to the N-terminus. The presence of a, b and y ions in combination enables the peptide to be sequenced initially in one direction, followed by confirmatory sequencing in the opposite direction. The presence of a, b and y ions in combination also allows the peptide to be sequenced initially using one ion type, and any ambiguities or gaps in the sequence to be filled by reference to the amino acid sequence derived from analysis of one or more of the other ion types. Thus, in the methods of the invention an amino acid sequence may be assigned to a peptide on the basis of the m/z separation of consecutive peaks in two or more of the a, b and y ion series. For example, an amino acid sequence might be assigned to a peptide following identification and analysis of the consecutive peaks in the a and b ion series, the a and y ion series or the b and y ion series. Preferably, an amino acid sequence is assigned to a peptide following identification and analysis of the consecutive peaks in the a, b and y ion series.

The invention also provides a method for determining an amino acid sequence of a peptide, comprising the steps of:
(a) obtaining a fragmentation mass spectrum for the peptide, wherein the fragmentation mass spectrum contains peaks corresponding to each of a, b and y daughter ions;
(b) identifying peaks in the fragmentation mass spectrum of step (a) corresponding to a, b or y daughter ions, preferably identifying peaks corresponding to a and b daughter ions, more preferably identifying peaks corresponding to a, b and y daughter ions; and
(c) assigning an amino acid sequence to the peptide on the basis of the m/z separation of consecutive peaks in one or more of the a, b and y ion series.

Proline is a unique amino acid because its side chain is bonded to the tertiary nitrogen in a cyclic pyrrolidine ring. In a hypothetical tripeptide (Xaa₁-Pro-Xaa₂), fragmentation due to cleavage of the amide bond at the N-terminal side of a proline residue (the Xaa₁-Pro bond) is thought to be dominant over fragmentation due to cleavage of the amide bond at the C-terminal side of a proline residue (the Pro-Xaa₂ bond). Accordingly, in known methods of fragmentation mass spectrometry it is usual to observe incomplete daughter ion series when the peptide contains a proline residue. In particular, it is usual that daughter ions generated by cleavage of the Xaa₁-Pro bond are detected whilst daughter ions generated by cleavage of the Pro-Xaa₂ bond are not detected, leaving a dipeptide gap in the ion series. Fragmentation of the peptide backbone at a proline residue may also be affected by the identity of the amino acid residues adjacent to the proline residue (Xaa₁ and Xaa₂). Accordingly, accurate sequence assignment to proline-containing peptides by fragmentation mass spectrometry remains technically challenging.

The inventors have also found that a peptide can be derivatised at its N-terminus such that fragmentation of the peptide backbone is not affected by the presence of a proline residue to the same extent as fragmentation of the underivatised peptide. Thus, the fragmentation mass spectra generated in the methods of the present invention may contain a pair of consecutive peaks in the a, b or y ion series (preferably a pair of consecutive peaks in the b or y ion series) with a m/z separation corresponding to a proline residue. Furthermore, the inventors have found that a peptide can be derivatised at its N-terminus such that fragmentation of the peptide backbone is not affected by the presence of multiple proline residues to the same extent as fragmentation of the underivatised peptide. Thus, the fragmentation mass spectra generated in the methods of the present invention may contain multiple pairs of consecutive peaks in the a, b or y ion series (preferably multiple pairs of consecutive peaks in the b or y ion series) with a m/z separation corresponding to a proline residue. Thus, a fragmentation mass spectrum of the derivatised peptide may contain additional information (relative to a fragmentation mass spectrum of an underivatised peptide) useful for determination of the amino acid sequence of the derivatised peptide.

The present invention thus provides a method for analysing a peptide by mass spectrometry, comprising the step of obtaining a fragmentation mass spectrum for the peptide, wherein the fragmentation mass spectrum contains a pair of consecutive peaks in the a, b or y ion series (preferably a pair of consecutive peaks in the b or y ion series), with a m/z separation corresponding to a proline residue.

Thus, the step of obtaining a fragmentation mass spectrum may comprise the steps of:
(a) reacting the peptide's N-terminus with a label to provide a derivatised peptide, wherein the peptide contains a proline residue and wherein the label stabilises daughter ions formed during fragmentation mass spectrometry and comprising a C-terminal proline residue and/or permits cleavage between the proline and the amino acid immediately C-terminal to it; and
(b) providing a fragmentation mass spectrum of the derivatised peptide, e.g. by fragmentation mass spectrometry (MSⁿ, wherein n is at least 2).

As noted above, the fragmentation mass spectrum of (b) contains a pair of consecutive peaks in the a, b or y ion series (preferably a pair of consecutive peaks in the b or y ion series) with a m/z separation corresponding to a proline residue.

For instance, this step (b) may comprise the steps of:
(b1) performing mass spectrometry of the derivatised peptide to obtain a first mass spectrum;
(b2) identifying at least one parent ion in the first mass spectrum;
(b3) selecting at least one parent ion for further analysis by mass spectrometry; and
(b4) performing fragmentation mass spectrometry of the selected parent ion(s) to obtain a fragmentation mass spectrum, wherein the fragmentation mass spectrum contains a pair of consecutive peaks in the a, b or y ion series, (preferably a pair of consecutive peaks in the b or y ion series) with a m/z separation corresponding to a proline residue.

The invention also provides a method for determining an amino acid sequence of a proline-containing peptide, comprising the steps of:
(a) obtaining a fragmentation mass spectrum for the peptide, wherein the fragmentation mass spectrum contains a pair of consecutive peaks in the a, b or y ion series (preferably a pair of consecutive peaks in the b or y ion series) with a m/z separation corresponding to a proline residue;
(b) analysing the mass spectrum to identify a pair of consecutive peaks in the a, b or y ion series (preferably in the b or y ion series), with a m/z separation corresponding to a proline residue; and
(c) assigning a proline residue to the peptide on the basis of a pair of consecutive peaks in the a, b or y ion series (preferably in the b or y ion series).

The methods of the present invention are also useful in determining an amino acid sequence of a peptide that cannot be directly sequenced by fragmentation mass spectrometry. In particular, the amino acid sequence of long peptides (i.e. polypeptides) may be identified by assembling the amino acid sequences of a number of peptides, as determined by fragmentation mass spectrometry, to provide the full-length amino acid sequence of the polypeptide. In such methods, analysis of first and second mixtures of peptides by fragmentation mass spectrometry may be required to provide the necessary information for assembly of the amino acid sequences of the peptides into the correct order. For example, first and second mixtures of peptides may be prepared by digestion of a polypeptide of interest with different protease enzymes.

The invention also provides a method for analysing a peptide by fragmentation mass spectrometry, comprising the steps of:
(a) reacting the peptide's N-terminus with a label to provide a derivatised peptide, wherein the label is an organic group that allows positive charge to be retained by a and b ions formed during fragmentation mass spectrometry of the derivatised peptide;
(b) ionising the derivatised peptide by MALDI or ESI; and
(c) performing fragmentation mass spectrometry of the ionised peptide;
   wherein a fragmentation mass spectrum generated in step (c) contains peaks corresponding to both a and b daughter ions.

The invention also provides a method for analysing a peptide by fragmentation mass spectrometry, comprising the steps of:
(a) reacting the peptide's N-terminus with a label to provide a derivatised peptide, wherein the label is an organic group that allows positive charge to be retained by a and b ions derived from fragmentation of the derivatised peptide; and
(b) analysing the derivatised peptide by MSⁿ, wherein n is at least 3;
wherein a fragmentation spectrum generated in step (b) contains peaks corresponding to both a and b daughter ions.

The invention also provides a method for analysing a peptide by fragmentation mass spectrometry, comprising the steps of:
(a) reacting the peptide's N-terminus with a label to provide a derivatised peptide, wherein the label is an organic group that allows positive charge to be retained by a and b ions derived from fragmentation of the derivatised peptide; and
(b) performing fragmentation mass spectrometry of the derivatised peptide, wherein CID or PSD is used to induce ion fragmentation;
wherein a spectrum generated in step (b) contains peaks corresponding to both a and b daughter ions.

The inventors have also found that a peptide can be derivatised at its N-terminus such that peaks corresponding to c and/or x and/or z ions are identifiable in fragmentation mass spectra of the derivatised peptide, in addition to peaks corresponding to a and b (and y) ions. As a result, the fragmentation mass spectra of the derivatised peptide may contain yet further additional information (relative to the fragmentation mass spectra of the underivatised peptide) useful for determination of the amino acid sequence of the peptide.

Thus, the invention also provides a method for analysing a peptide by mass spectrometry, comprising the step of obtaining a fragmentation mass spectrum for the peptide, wherein the fragmentation mass spectrum contains peaks corresponding to c and/or x and/or z ions, in addition to peaks corresponding to a and b (and y) ions. The methods of the invention may include identifying peaks in a fragmentation mass spectrum that correspond to c and/or x and/or z ions, in addition to peaks corresponding to a and b (and y) ions. The peptide may be derivatised as described above.

### The Sample

The term "peptide" includes any molecule comprising two or more amino acids joined to each other by peptide bonds or modified peptide bonds, *i.e*. peptide isosteres. This term refers both to short chains (*e.g.* oligopeptides with fewer than 20 amino acids) and to longer chains (*e*.*g*. polypeptides with 20 or more amino acids).

The present invention is not limited to fragmentation mass spectrometry of short peptides and polypeptides. For example, fragmentation mass spectrometry analysis of whole proteins by "top-down" sequencing is also possible. Thus, the term "peptide" used herein can also encompass whole proteins.

The peptide analysed by the methods of the invention will be within a sample, and that sample may comprise a single peptide or a mixture of different peptides.

The peptide may be a linear, cyclic or branched peptide. The peptide will generally have a free N-terminus for derivatisation of the peptide with a suitable label. Preferably, the peptide is a linear peptide.

The peptides may contain either L- and/or D- amino acids. Preferably, the peptides contain L- amino acids only (including glycine).

The peptides may contain amino acids other than the 20 'classical' gene-encoded amino acids. For example, the peptides may contain amino acids incorporated directly by an unusual mRNA translation step (*e.g*. selenocysteine). The peptides may also contain amino acids produced by metabolic conversions of free amino acids (*e.g.* ornithine and citrulline). The peptides may also contain amino acids that include post-transitional modifications (*e.g.* acetylation, amidation, deamidation, biotinylation, C-mannosylation, flavinylation, farnesylation, formylation, geranyl-geranylation, lipidation, phosphorylation, glycosylation, hydroxylation, disulphide bond formation, methylation, myristoylation, sulphation, carboxylation, ADP-ribosylation, *etc*.). The peptides may also contain amino acids that have been modified by chemical modification techniques, which are well known in the art. Typically, the term "proline" also includes hydroxyproline.

The modifications that occur in a peptide often will be a function of how the peptide is made. For peptides that are made recombinantly, the nature and extent of the modifications in large part will be determined by the post-translational modification capacity of the particular host cell and the modification signals that are present in the amino acid sequence of the peptide in question. For instance, glycosylation patterns vary between different types of host cell.

Modifications can occur anywhere in the peptide, including the peptide backbone, the amino acid side-chains and the amino or carboxyl termini. Blockage of the amino or carboxyl terminus in a peptide by a covalent modification is common in naturally-occurring and synthetic polypeptides and such modifications may be present in the peptides.

The peptides can be prepared in any suitable manner. For example, the peptides may be prepared biologically (for example, by culture of naturally-occurring or recombinant cell types), or may be prepared synthetically (for example, by chemical synthesis).

A mixture of peptides includes 2 or more different peptides, *e.g.* >5 peptides, > 10 peptides, >20 peptides, >30 peptides, >40 peptides, >50 peptides, >60 peptides, >70, peptides, >80 peptides, >90 peptides, >100 peptides, *etc.* Peptide mixtures can be prepared in any suitable manner. For example, the mixture of peptides may be prepared directly from a cell type of interest (its proteome in whole or part), or may be prepared by cleavage of one or more polypeptides. Polypeptide cleavage may be enzymatic or non-enzymatic. Suitable enzymatic reagents include, but are not limited to, Trypsin, Arg-C, Asp-N, Asp-N-ambic, chymotrypsin, Lys-C, Lys-C/P, PepsinA, S. Aureus pH 4, S. Aureus pH 8, Pancreatic Elastase, Thermolysin, Clostripain, V8-DE, V8-E, Thrombin, Factor Xa Protease, Enterokinase, endopeptidase rTEV from tobacco etch virus, 3C human rhinovirus protease, *etc.* Suitable non-enzymatic cleavage reagents include, but are not limited to, CNBr, Formic acid, Hydroxylamine, *etc.*

Preferably, a mixture of peptides is prepared by digesting one or more polypeptides with a protease. Preferably, the protease enzyme is selected for its cleavage specificity. Enzymes that cleave polypeptides indiscriminately will lead to a mixture of peptides producing a complex mass spectrum. Conversely, enzymes that cleave only at very rare positions will lead to a mixture of peptides producing a simple mass spectrum from which it may not be possible to select a suitable number of parent ions. Examples of commonly used protease enzymes are given in the previous paragraph.

Where the experimental protocol involves initial separation of an individual peptide in a sample from other peptides (for example, by 2D SDS-PAGE), preparation of the mixture of peptides by digestion of a polypeptide with a protease may be carried out *in situ* in the separation medium.

Within a sample, a peptide may be free in solution or, as an alternative, may be attached to a solid support, covalently or non-covalently. Where the peptide is attached to a solid support, it will be removed from the solid support for analysis by fragmentation mass spectrometry.

In addition to the peptide(s), the sample may also include one or more solvents, one or more buffers, one or more salts, one or more detergents, one or more protease inhibitors, *etc.*

### Derivatisation with label

Peptide(s) within a sample are reacted with a label to provide derivatised peptides for fragmentation mass spectrometry. Derivatisation with an organic group that allows positive charge to be retained by a and b ions formed during fragmentation mass spectrometry of the derivatised peptide gives derivatised peptides that, when analysed by tandem mass spectrometry, give rise to spectra that contain peaks corresponding to both a and b daughter ions. This is because a and b fragments formed during fragmentation mass spectrometry of underivatised peptides by known methods are usually uncharged and are therefore not detected.

As well as providing derivatised peptides that, when analysed by fragmentation mass spectrometry, give rise to spectra that contain peaks corresponding to both a and b daughter ions, advantageous labels can give derivatised peptides that, when analysed by fragmentation mass spectrometry, give rise to spectra that contain peaks corresponding to each of a, b and y daughter ions.

As well as providing derivatised peptides that, when analysed by fragmentation mass spectrometry, give rise to spectra that contain peaks corresponding to a and b (and y) daughter ions, advantageous labels can give derivatised peptides that, when analysed by fragmentation mass spectrometry, give rise to spectra that contain peaks corresponding to c and/or x and/or z ions, in addition to peaks corresponding to a and b (and y) ions.

As well as providing derivatised peptides that, when analysed by fragmentation mass spectrometry, give rise to spectra that contain peaks corresponding to a, b and y daughter ions, advantageous labels can improve the ionisation properties of the peptide.

As well as providing derivatised peptides that, when analysed by fragmentation mass spectrometry, give rise to spectra that contain peaks corresponding to each of a, b and y daughter ions, advantageous labels allow fragmentation of the peptide backbone during fragmentation mass spectrometry despite the presence of one or more proline residues in the peptide.

One such class of labels is trityl derivatives, as disclosed in EP 1506959 A2. Preferred labels have formulae (IIa), (IIb), (IVai), (IVaii), (IVaiii), (IVbii), (IVbiii), (IVaiv) and (IVbiv), as defined in EP 1506959 A2. More preferably, the labels have formulae (IIa), (IIb), (IVai), (IVaii), (IVaiii), (IVbii), (IVbiii), (IVaiv) and (IVbiv), as defined in UK patent application 0328414.8:

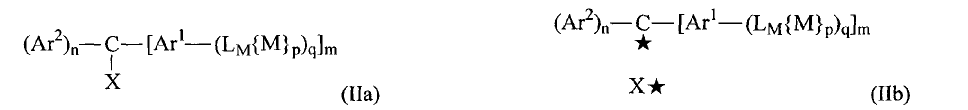

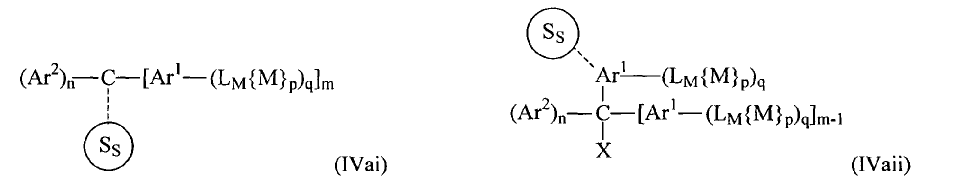

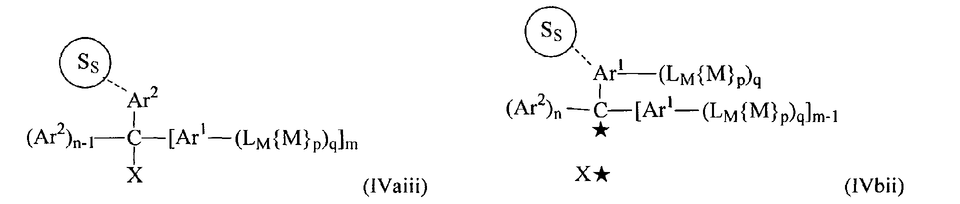

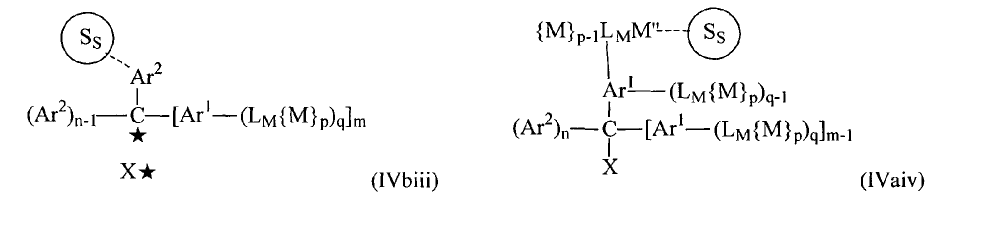

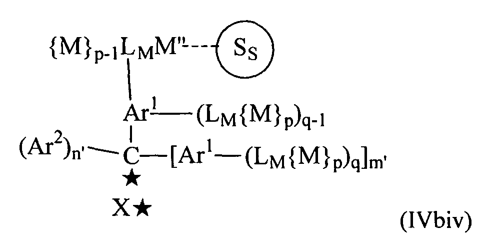

Preferred features of these formulae as disclosed in EP 1506959 A2 or GB 0328414.8 are also preferred features of labels for use with this invention.

As described above, the label allows positive charge to be retained by daughter ions formed during fragmentation mass spectrometry of the derivatised peptide. Thus, the label increases the intensity of the daughter ions in the fragmentation mass spectra by allowing them to carry a fixed positive charge. In addition, the label may advantageously allow the daughter ions to exist as radical ions.

In order to react with a peptide, the label may be free in solution (*e.g.* the label can be added to the peptide and reacted in solution) or, as an alternative, may be attached (covalently or non-covalently) to a solid support (*e.g.* peptides can be added to immobilised label and subsequently released from the support for analysis by mass spectrometry).

The reaction may be carried out at any stage prior to analysis of the peptide(s) by fragmentation mass spectrometry. For example, the reaction may be carried out before separation of the polypeptides in a sample. As an alternative, the reaction may be carried out following separation of the polypeptides in a sample but before digestion of one or more individual polypeptides. As a further alternative, the reaction may be carried out following digestion of a polypeptide of interest to provide a mixture of peptides. Labelling before digestion gives fewer labels per original polypeptide sequence than labelling after digestion.

It is preferred that the peptides(s) in a peptide mixture are derivatised, *i.e.* the reaction is preferably carried out following digestion of a polypeptide of interest.

The derivatisation reaction may proceed directly or indirectly. For example, a group present on the peptide may react directly with a group on the label. Alternatively, the peptide may initially be derivatised with one or more suitable groups (*e.g.* N-hydroxysuccinimide) for subsequent reaction with a suitable group on the label. Thus, the present invention allows one or more steps of peptide manipulation prior to derivatisation with the label.

It is possible for a peptide to be labelled by two separate labels (*e.g.* one at the N-terminus and one on a lysine side chain). Double labelling may be seen, for instance, when a peptide has a C-terminal lysine residue, *e.g.* after trypsin digestion. Thus peptides of the invention may carry one or more labels (e.g. 2, 3, 4, 5 or more).

The invention also provides a method for screening for a label that allows positive charge to be retained by a and b ions formed during fragmentation mass spectrometry of a peptide derivatised with the label, comprising the steps of:
(a) obtaining a candidate label;
(b) reacting the candidate label with a peptide to provide a derivatised peptide;
(c) performing fragmentation mass spectrometry of the derivatised peptide; and
(d) analysing a fragmentation mass spectrum generated in step (c) to determine if it contains peaks corresponding to both a and b ions.

If a spectrum contains peaks corresponding to both a and b ions derived from fragmentation of the peptide (*e.g.* after deisotoping) then the candidate label is a label suitable for use with the invention.

The invention also provides a method for screening for a label that stabilises daughter ions formed during fragmentation mass spectrometry and comprising a C-terminal proline residue and/or permits cleavage between the proline and the amino acid immediately C-terminal to it, comprising the steps of:
(a) obtaining a candidate label;
(b) reacting the candidate label with a peptide to provide a derivatised peptide;
(c) performing fragmentation mass spectrometry of the derivatised peptide; and
(d) analysing a fragmentation mass spectrum generated in step (c) to determine if it contains a pair of consecutive peaks in the a, b or y ion series, preferably in the b or y ion series, with a m/z separation corresponding to a proline residue.

If a spectrum contains a pair of consecutive peaks in the a, b or y ion series (preferably in the b or y ion series) with a m/z separation corresponding to a proline residue (*e.g.* after deisotoping) then the candidate label is a label suitable for use with the invention.

Candidate labels may be derived from large libraries of synthetic or natural compounds. For instance, synthetic compound libraries are commercially available from MayBridge Chemical Co. (Revillet, Cornwall, UK) or Aldrich (Milwaukee, WI). Alternatively, libraries of natural compounds in the form of bacterial, fungal, plant and animal extracts may be used. Additionally, candidate labels may be synthetically produced using combinatorial chemistry either as individual compounds or as mixtures.

### Derivatised Peptides

The invention provides a peptide with a N-terminal residue, characterised in that (a) a label that allows positive charge to be retained by a and b ions formed during fragmentation mass spectrometry of the peptide is attached to the N-terminal residue of the peptide and (b) the peptide can form both a and b daughter ions when analysed by fragmentation mass spectrometry.

The invention also provides a peptide with a N-terminal residue, characterised in that (a) a label that allows positive charge to be retained by a and b ions formed during fragmentation mass spectrometry of the peptide is attached to the N-terminal residue of the peptide and (b) the peptide provides a fragmentation mass spectrum that contains at least one a/b doublet. Preferably, the peptide provides a fragmentation mass spectrum that contains at least 30% (e.g. 40%, 50%, 60%, 70%, 80%, 90% or even 100%) of the theoretical a/b doublets of the peptide. Preferably, the peptide provides a fragmentation mass spectrum including a/b doublets for at least 30% (e.g. 40%, 50%, 60%, 70%, 80%, 90% or even 100%) of the peptide's amino acid residues.

The inventors have found that a peptide can be derivatised at its N-terminus such that fragmentation of the peptide is not affected by the presence of a proline residue to the same extent as fragmentation of the underivatised peptide. Thus, the fragmentation mass spectra generated in the methods of the present invention may contain a pair of consecutive peaks in the a, b or y ion series (preferably a pair of consecutive peaks in the b or y ion series) with a m/z separation corresponding to a proline residue. Furthermore, the inventors have found that a peptide can be derivatised at its N-terminus such that fragmentation of the peptide backbone is not affected by the presence of multiple proline residues to the same extent as fragmentation of the underivatised peptide. Thus, the fragmentation mass spectra generated in the methods of the present invention may contain multiple pairs of consecutive peaks in the a, b or y ion series (preferably multiple pairs of consecutive peaks in the b or y ion series) with a m/z separation corresponding to a proline residue.

The invention also provides a peptide having a N-terminal residue and including a proline residue downstream of the N-terminal residue, characterised in that (a) a label that stabilises daughter ions formed during fragmentation mass spectrometry and comprising a C-terminal proline residue and/or permits cleavage between the proline and the amino acid immediately C-terminal to it is attached to the N-terminal residue of the peptide and (b) the peptide provides a fragmentation mass spectrum that contains a pair of consecutive peaks in the a, b or y ion series (preferably in the b or y ion series) with a m/z separation corresponding to a proline residue. The N-terminus amino acid can be any amino acid, including proline. In some embodiments, peptides consisting of the amino acid sequence NH₂-RHPEYAVSVLLR-COOH (SEQ ID NO:1) or NH₂-DDPHACYSTVFDK-COOH (SEQ ID NO:2) are specifically excluded from the invention.

Preferably the C-terminal residue of the peptides of the invention is lysine or arginine.

Preferably, the peptide comprises at least A amino acids, where A is 2 or more (*e.g.* 2, 3, 4, 5, 6, 7, 8, 9, 10, 11, 12, 13, 14, 15, 16, 17, 18, 19, 20, 21, 22, 23, 24, 25, 26, 27, 28, 29, or 30). Preferably, the peptide comprises at most B amino acids, where B is 2000 or less (*e.g.* 2000, 1500, 1000, 500, 475, 450, 425, 400, 375, 350, 325, 300, 275, 250, 225, 200, 190, 180, 170, 160, 150, 140, 130, 120, 110, 100, 99, 98, 97, 96, 95, 94, 93, 92, 91, 90, 89, 88, 87, 86, 85, 84, 83, 82, 81, 80, 79, 78, 77, 76, 75, 74, 73, 72, 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31 or 30).

The present invention also provides ionic forms of the peptides of the invention, protonated ionic forms of the peptides of the invention, free radical forms of the peptides of the invention and free radical ionic forms of the peptides of the invention. Preferably, the ionic forms are cationic.

Free radical ions, in particular cations, are the most preferred ions for use with the invention.

The present invention also provides a mixture of these forms of the peptides. A mixture of these forms of the peptides includes 2 or more different peptides, *e*.*g*. >5 peptides, > 10 peptides, >20 peptides, >30 peptides, >40 peptides, >50 peptides, >60 peptides, >70, peptides, >80 peptides, >90 peptides, >100 peptides, *etc.* The peptides in the mixture may each independently be present as an ionic form, a protonated ionic, a free radical form or a free radical ionic form.

The methods of the invention may involve use of a kit comprising: (a) a label for derivatisation of peptide(s) to provide derivatised peptides, wherein the label is a label that allows positive charge to be retained by a and b ions formed during fragmentation mass spectrometry of a derivatised peptide; and (b) one or more other components selected from the group consisting of: a separation medium (*e.g.* an electrophoresis gel or chromatography column), a protease, a protease inhibitor, a solvent, a buffer, a salt, a detergent, a mass standard and a matrix compound.

The methods of the invention may involve use of a kit comprising: (a) a label for derivatisation of peptide(s) to provide derivatised peptides, wherein the label is a label that stabilises daughter ions comprising a C-terminal proline residue and/or permits cleavage between the proline and the amino acid immediately C-terminal to it; and (b) one or more other components selected from the group consisting of: a separation medium (*e.g.* an electrophoresis gel or chromatography column), a protease, a protease inhibitor, a solvent, a buffer, a salt, a detergent, a mass standard and a matrix compound.

### Fragmentation Mass Spectrometry

As described above, the step of obtaining a fragmentation mass spectrum may comprise providing a fragmentation mass spectrum of a derivatised peptide by MSⁿ, wherein n is at least 2. Preferably, n is 2.

As described above, the step of providing a fragmentation mass spectrum of a derivatised peptide by MSⁿ, wherein n is at least 2, may comprise the steps of:
(a) performing mass spectrometry of the derivatised peptide to obtain a first mass spectrum;
(b) identifying at least one parent ion in the first mass spectrum;
(c) selecting at least one parent ion for further analysis by mass spectrometry; and
(d) performing fragmentation mass spectrometry of the selected parent ion(s) to obtain a fragmentation mass spectrum, wherein the fragmentation mass spectrum contains peaks corresponding to both a and b ions derived from fragmentation of the derivatised peptide.

As described above, the step of performing fragmentation mass spectrometry of the selected parent ion(s) to obtain a fragmentation mass spectrum may provide a fragmentation mass spectrum that contains peaks corresponding to each of a, b and y ions derived from fragmentation of the derivatised peptide.

In preferred embodiments of the invention, at least 50% (e.g. 60%, 70%, 80%, 90% or even 100%) of the theoretical a, b and/or y daughter ions of a given peptide are detected.

In preferred embodiments, at least 30% (e.g. 40%, 50%, 60%, 70%, 80%, 90% or even 100% of the theoretical a/b doublets of a given peptide are detected.

Various methods are known in the art for selecting parent ions for further analysis by fragmentation mass spectrometry, and those methods may be used in the methods of the invention.

In the methods of the invention, the parent ion selection step (iii) may be performed on the basis of parent ion charge, parent ion mass, parent ion mass/charge ratio or parent ion abundance. Preferably, the selection step is performed on the basis of a combination of parent ion mass/charge ratio and abundance. More preferably, at least one doubly charged parent ion is selected (e.g. in ESI). The skilled person should determine a suitable parent ion selection method and a suitable width of selection window in light of the quality of the sample and the mass spectrometer, and the ions that they wish to select. For example, the skilled person might choose a selection window to encompass all isotopic variants of a given parent ion or only those parent ions having the monoisotopic mass.

The mass spectrometer may comprise any of a number of combinations of ion source, mass analyser and ion detector.

Suitable ion sources include, but are not limited to, matrix-assisted laser desorption ionisation (MALDI), secondary ion mass spectrometry (SIMS), fast atom bombardment (FAB) and electrospray ionisation (ESI) ion sources. Preferably, the ion source is a MALDI ion source or an ESI ion source. The MALDI ion source may be traditional MALDI source (under vacuum) or may be an atmospheric pressure MALDI (AP-MALDI) source.

Suitable mass analysers include, but are not limited to, time of flight (TOF), quadrupole time of flight (Q-TOF), ion trap (IT), quadrupole ion trap (Q-IT), triple quadrupole (QQQ), Fourier transform mass spectrometry (FTMS) and Fourier transform ion cyclotron resonance (FTICR) mass analysers. Preferably, the mass analyser is a TOF mass analyser. TOF analysers generally include a reflectron.

Preferably, the mass spectrometer is an ESI-Q-TOF mass spectrometer or a MALDI-Q-TOF mass spectrometer.

FTICR-MS and MALDI-TOF-TOF-MS methods are particularly useful for "top-down" sequencing of whole proteins without digestion.

Fragmentation mass spectrometry will include the step of inducing fragmentation of the selected parent ion(s) to produce daughter ions. The fragmentation of the parent ion(s) to produce daughter ions may be effected by, for example, collision induced dissociation (CID), surface induced dissociation (SID), infrared multiphoton dissociation (IRMPD), blackbody multiphoton dissociation (BIRD), sustained off-resonance irradiation (SORI), electron capture dissociation (ECD) or post source decay (PSD). Preferably, the fragmentation of the parent ion to produce daughter ions is induced by CID or PSD.

Mass spectrometry of the derivatised peptide(s) by fragmentation mass spectrometry includes multiple ion detection steps. The mass analyser need not be the same for each data collection step. The mass spectrometer may be a tandem-in-space mass spectrometer, wherein the different stages of mass analysis are performed sequentially in discrete regions of the instrument. Alternatively, the mass spectrometer may be a tandem-in-time mass spectrometer, wherein the different stages of mass analysis are performed in the same region of the instrument at different times.

For MALDI-MS, a sample containing a peptide is mixed with a matrix compound prior to spotting onto a target plate. The matrix compound is selected such that it absorbs the wavelength of laser light which is to be used for ionisation, is able to co-crystallise with the peptide(s), is vacuum stable, causes desorption of the peptide(s) upon laser irradiation and promotes peptide ionisation. A wide variety of matrix compounds useful for peptides are known in the art, including alpha-cyano-4-hydroxycinnamic acid (CHCA), sinapic acid (SA), 2-(4-hydroxyphenylazo)benzoic acid (HABA), 2,5-dihydroxybenzoic acid, 2,4,6- and 2,3,4-trihydroxyacetonephenone, succinic acid, 2,6-dihydroxyacetophenone, ferulic acid, caffeic acid, glycerol and 4-nitroaniline. The methods of the invention therefore also involve a mixture of a derivatised peptide of the invention and a matrix compound.

For ESI-MS, a sample containing a peptide is mixed with an organic solvent (*e.g.* methanol, acetonitrile, ethanol), water and an acid for positive ion mass spectrometry (*e.g.* acetic acid, formic acid) or a base for negative ion mass spectrometry (*e.g.* ammonium salts). The methods of the invention therefore also involve a mixture of a derivatised peptide and an organic solvent, water and an acid or base.

As noted above, the reaction of a label with peptide(s) within a sample may be carried out at any stage prior to analysis of the peptide(s) by mass spectrometry. The reaction may be carried out after or, preferably, before mixing the peptide(s) with the matrix compound.

Mass spectrometry of the derivatised peptide(s) may include the analysis of mass standards added to the sample prior to mass spectrometry. Alternatively, one or more components already present in the sample may be used as a mass standard. For example, autoproteolytic fragments of a protease used to produce a peptide mixture are often used as mass standards.

The present invention also provides derivatised a and b daughter ions produced during the methods of the present invention, wherein the derivatised a and b daughter ions comprise a C-terminal proline residue.

The present invention also provides a mass spectrometer comprising an ion source, a mass analyser and an ion detector, wherein the mass spectrometer contains derivatised a and b daughter ions of the invention. The mass spectrometer may also contain y daughter ions.

The present invention also provides a mass spectrometer collision cell, wherein the collision cell contains derivatised a and b daughter ions of the invention. The mass spectrometer may also contain y daughter ions.

The present invention also provides a mass spectrometer ion trap, wherein the collision cell contains derivatised a and b daughter ions of the invention. The mass spectrometer may also contain y daughter ions.

### Analysis of Mass Spectrometry Data

Analysis of the fragmentation mass spectra generated in the methods of the present invention may be performed using known methods. As described above, an amino acid sequence may be assigned to a peptide on the basis of the m/z separation of consecutive peaks in two or more of the a, b and y ion series.

The analysis of the mass spectra may be carried out manually or may be automated. Preferably, the analysis of the mass spectra is automated, for example by using a computer.

The initial analysis of raw mass spectra may include deisotoping and/or identification of the monoisotopic masses of the ions. The existence of an isotopic distribution in the mass spectrum of peptide ions is well known in the art. The isotopic distribution for a peptide is dictated by the relative natural abundance of the isotopes of the elements present in the peptide, and all peptides normally display a similar isotopic distribution pattern. Modem mass spectrometers are capable of resolving the isotopic distribution of individual molecules, by separating ions containing ¹²C, ¹H and ¹⁶O from ions of the same molecule that contain one or more atoms of ¹³C, ²H or ¹⁷O. Thus, modern mass spectrometers are not limited to a determination of the average ion mass. Deisotoping of the mass spectrum is used to identify the monoisotopic mass for a peptide from the isotopic distribution pattern present in the mass spectrum. The monoisotopic mass of a peptide ion is the mass of the lightest ion for that peptide (*i.e.* the mass of the ion that contains the lightest isotope of each of the elements that contribute to the isotopic distribution). Various computer algorithms are known in the art for deisotoping mass spectra (*e.g.* 'Collapse', produced by Positive Probability Ltd). Deisotoping the mass spectrum is generally preceded by centroiding the peaks within each isotopic distribution to provide a number of defined peaks for each peptide. The pattern of centroided peaks is then deisotoped by comparison of the measured intensities of the peaks in each cluster against the intensities of peaks within generic template isotopic distributions for peptides.

The initial analysis of the mass spectrum of the derivatised peptide(s) may also include the identification of the relative intensity of the peaks generated by each isotope.

There are a number of computer packages available for the automated identification of monoisotopic masses of peptides from the mass spectrum and the intensities of the peaks within the isotopic distribution for each peptide. Suitable computer packages include, but are not limited to MASCOT and PROWL.

There are a number of computer packages available for the automated analysis of fragmentation mass spectra. For example, all the mass spectrometer manufacturers have their own programs for analysing fragmentation data, as well as the search engine providers such as MASCOT. Suitable computer packages include, but are not limited to, BioLynx and Analyst. The program should permit the N-terminal modification of the sample to be specified.

As described above, the presence of peaks corresponding to a, b and y ions in the mass spectra of daughter ions derived from fragmentation of derivatised peptides facilitates the assignment of amino acid sequence information to the parent ion, due to the large amount of sequence-specific information provided by those peaks. In particular, the presence of a/b doublets in the fragmentation mass spectra generated in the methods of the invention enables rapid assignment of a and b ion types to the peaks in the fragmentation mass spectra (the a ion of each a/b doublet will have the lower m/z). This additional information provides an improvement in the accuracy of results returned by such computer packages, and may allow unambiguous determination of an amino acid sequence for the parent ion.

Automated analysis of the fragmentation mass spectra generated in the methods of the invention will take into account the mass of the label located at the N-terminus of the peptide. In the methods of the invention, the a and b ions will retain the label. Accordingly, the observed m/z for the a and b daughter ions must be adjusted to take account of the (known) mass of the label, to arrive at the actual m/z for the relevant ion. In contrast, the observed m/z for the y daughter ions need not be adjusted to take account of the mass of the label. This type of information can be specified in known computer packages.

The inventors have also found that peptides can be derivatised such that, when ionised and analysed by mass spectrometry, those peptides containing arginine residues give characteristic peak patterns. Peaks corresponding to arginine-containing peptides can therefore be located in a mass spectrum in order to simplify and improve peptide analysis. Suitable labels for discrimination of arginine-containing peptides are those that give derivatised peptides that have the ability to form both a stabilised ion species ([P]⁺) and a protonated ion molecular species ([P+H]⁺) that differ by one average mass unit. Such labels include labels of the present invention, which are organic groups that allow a positive charge to be retained by a and b ions formed during fragmentation mass spectrometry of the derivatised peptide. Because derivatised arginine-containing peptides can form these two different species, a characteristic peak pattern is seen for those peptides, in which the stabilised ion species ([P]⁺) is less abundant than the protonated ion molecular species ([P+H]⁺). This peak pattern is not seen for derivatised peptides that do not contain arginine residues.

Thus, the methods of the present invention may further comprise the step of analysing a fragmentation mass spectrum to determine if it contains a peak pattern for an a or b ion in which a first peak and a second peak are separated by one average mass unit and in which the first peak is less abundant than the second peak and has a lower mass than the second peak. Preferably, the spectrum analysed in this embodiment is a deisotoped spectrum and the first peak and second peak are monoisotopic mass peaks.

### Database Searching

The amino acid sequence information derived from analysis of fragmentation mass spectra of derivatised peptides may be also used in database searching methods to identify a polypeptide of interest from which the derivatised peptides were derived.

In some embodiments, the amino acid sequence information alone may be used to identify the polypeptide of interest.

Thus, the invention provides a method for identifying a polypeptide by mass spectrometry, comprising the steps of:
(a) obtaining a fragmentation mass spectrum of a peptide derived from a polypeptide, wherein the mass spectrum contains peaks corresponding to a and b ions derived from fragmentation of the peptide;
(b) identifying peaks in the fragmentation mass spectrum of step (a) corresponding to a or b daughter ions, preferably identifying peaks corresponding to both a and b daughter ions; and
(c) assigning an amino acid sequence to the peptide on the basis of the m/z separation of consecutive peaks in the a or b ion series; and
(d) searching a database using amino acid sequence information generated in step (c) to identify the polypeptide.

The invention also provides a method for identifying a polypeptide by mass spectrometry, comprising the steps of:
(a) obtaining a fragmentation mass spectrum of a peptide derived from a polypeptide, wherein the mass spectrum contains peaks corresponding to a, b and y ions derived from fragmentation of the peptide;
(b) identifying peaks in the fragmentation mass spectrum of step (a) corresponding to a, b or y daughter ions, preferably identifying peaks corresponding to a and b daughter ions, more preferably identifying peaks corresponding to a, b and y daughter ions; and
(c) assigning an amino acid sequence to the peptide on the basis of the m/z separation of consecutive peaks in one or more of the a, b and y ion series; and
(d) searching a database using amino acid sequence information generated in step (c) to identify the polypeptide.

In alternative embodiments, amino acid sequence information may be combined with a peptide mass fingerprint for a polypeptide of interest. Thus, the invention enables simplification of the sequence space that needs to be searched for each peptide, by searching a database containing only sequences that contain the relevant amino acid sequence.

Thus, the invention provides a method for identifying a polypeptide by mass spectrometry, comprising the steps of:
(a) obtaining a first mass spectrum of a mixture of peptides derived from a polypeptide;
(b) identifying monoisotopic masses of the peptides in the first mass spectrum;
(c) selecting one or more parent ions for further analysis by mass spectrometry;
(d) obtaining a fragmentation mass spectrum for the parent ion(s) selected in step (c), wherein the fragmentation mass spectrum contains peaks corresponding to a and b ions;
(d) identifying peaks in the fragmentation mass spectrum of step (c) corresponding to a or b ions, preferably identifying peaks corresponding to a and b ions;
(e) assigning an amino acid sequence to the selected parent ion(s) on the basis of the m/z separation of consecutive peaks in the a or b ion series; and
(f) searching a database using information generated in steps (b) and (e) to identify the polypeptide.

As described above, an amino acid sequence may be assigned to a peptide on the basis of the m/z separation of consecutive peaks in the a and/or b ion series.

The invention also provides a method for identifying a polypeptide by mass spectrometry, comprising the steps of:
(a) obtaining a first mass spectrum of a mixture of peptides derived from a polypeptide;
(b) identifying monoisotopic masses of the peptides in the first mass spectrum;
(c) selecting one or more parent ions for further analysis by mass spectrometry;
(d) obtaining a fragmentation mass spectrum for the parent ion(s) selected in step (c),
   wherein the fragmentation mass spectrum contains peaks corresponding to a, b and y ions;
(d) identifying peaks in the fragmentation mass spectrum of step (c) corresponding to a, b or y ions, preferably identifying peaks corresponding to a and b ions, more preferably identifying peaks corresponding to a, b and y ions;
(e) assigning an amino acid sequence to the selected parent ion(s) on the basis of the m/z separation of consecutive peaks in one or more of the a, b and y ion series; and
(f) searching a database using information generated in steps (b) and (e) to identify the polypeptide.

As described above, an amino acid sequence may be assigned to a peptide on the basis of the m/z separation of consecutive peaks in two or more of the a, b and y ion series.

Database searching may be carried out using any suitable computer package.

### BRIEF DESCRIPTION OF THE FIGURES

Figure 1 shows the daughter ion types generated by backbone fragmentation of a hypothetical tripeptide.
Figure 2 shows the ESI-Q-TOF mass spectrum of purified dimethoxytrityl-labelled GluFib B peptide.
Figure 3 shows the ESI-Q-TOF-CID-MS/MS spectrum (Figure 3a) and the MALDI-Q-TOF-CID-MS/MS spectrum (Figure 3b) of the peptide NH₂-EALDFFAR-COOH (SEQ ID NO:3).
Figure 4 shows the ESI-Q-TOF-MS/MS mass spectrum of GluFib B peptide (Figure 4a) without and (Figure 4b) with b and y ion series marked.
Figure 5 shows the daughter ion types generated by backbone fragmentation of the GluFib B peptide whose mass spectrum is shown in Figure 4, alongside the daughter ion types normally observed in the mass spectrum of the same peptide when underivatised.
Figure 6 shows the MALDI-Q-TOF-CID-MS/MS mass spectrum of NH₂-GPFPIIV-COOH (SEQ ID NO:4), a proline-containing peptide.

### EXAMPLES

In the following examples, fragmentation mass spectrometry of peptides derivatised with a dimethoxytrityl label (in particular, a 4,4'-dimethoxy-4"-carboxyethyl trityl label) was performed.

### Example 1: Predominant formation of doubly charged molecular ion

As illustrated by Figure 1, the labels of the invention lead to predominant formation of doubly charged peptide ions in ESI-MS. Doubly charged ions are the preferred ions for fragmentation mass spectrometry, since they allow both N- and C- terminal daughter ions to be detected.

### Example 2: Identification of a, b and y ions in MSⁿ spectra of daughter ions derived from fragmentation of derivatised peptides

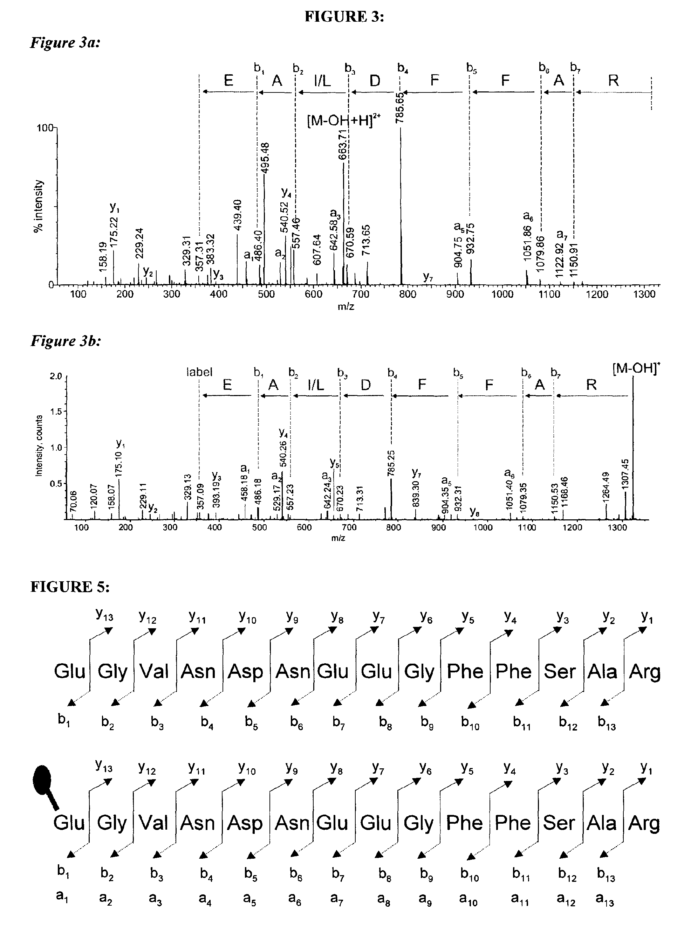

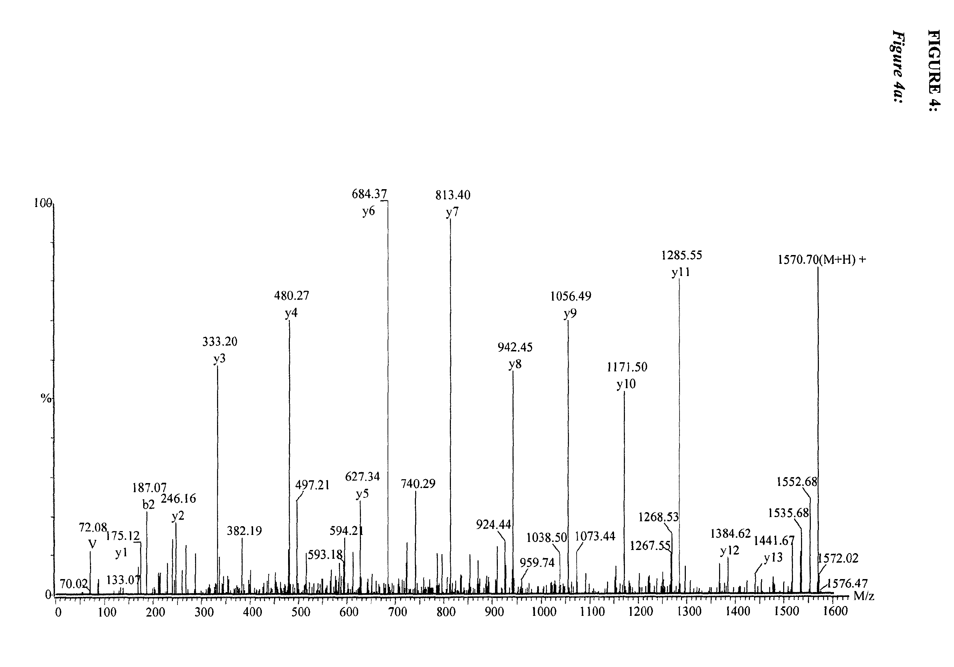

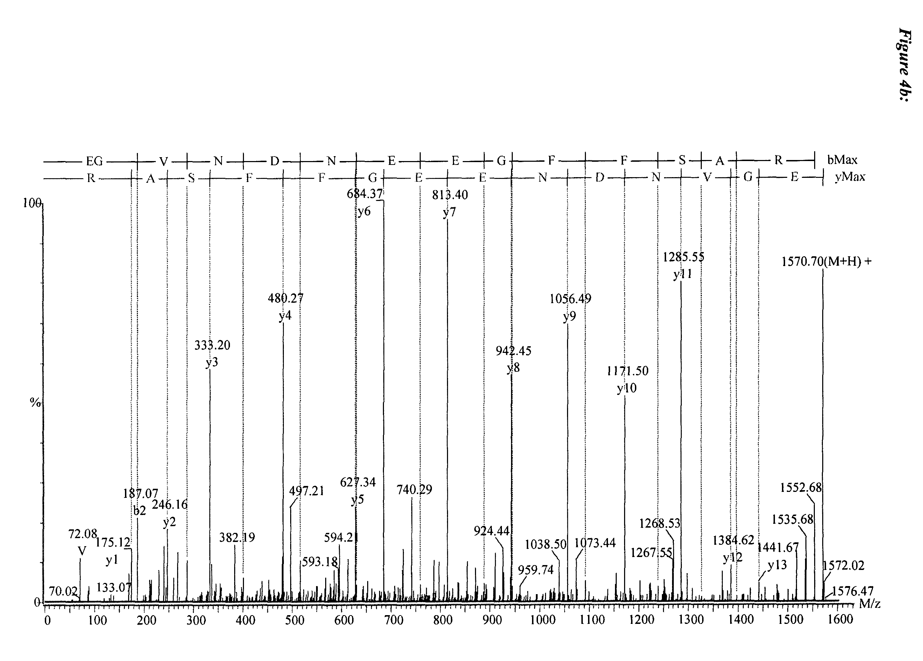

Figure 3 shows the ESI-Q-TOF-CID-MS/MS spectrum (Figure 3a) and the MALDI-Q-TOF-CID-MS/MS spectrum (Figure 3b) of the same peptide NH₂-EALDFFAR-COOH (SEQ ID NO:3).

The parent ion selected for fragmentation mass spectrometry is highlighted. The parent ion selected in the ESI experiment is doubly charged, whereas that selected in the ESI experiment is singly charged.

In Figure 3a, a complete b ion series (b₁-b₇) is visible and an almost complete a ion series (a₁, a₂, a₃, a₅, a₆ and a₇) is visible. In addition, a number of y ions are visible in the mass spectra of Figure 3a. Accordingly, the mass spectrum shown in Figure 3a permits rapid and simple assignment of an amino acid sequence to the peptide. Similarly, in Figure 3b, a complete b ion series (b₁-b₇) is visible and an almost complete a ion series (a₁, a₂, a₃, a₅ and a₆) is visible. In addition, a number of y ions are visible in the mass spectra of Figure 3b. Accordingly, the mass spectrum shown in Figure 3b also permits rapid and simple assignment of an amino acid sequence to the peptide. Thus, it is clear from Figure 3 that the fragmentation pattern for both ESI and MALDI is similar with respect to the presence of both a and b daughter ions in the mass spectrum.

As noted above, the fragmentation mass spectra generated in the methods of the present invention comprise a number of aₙ and bₙ ion peak pairs, wherein a peak corresponding to an aₙ ion is separated from a peak corresponding to a bₙ ion by the m/z of a carbonyl group (28 Da/z). Figure 3 therefore also highlights the manner in which the presence of a series a/b doublets in the fragmentation mass spectra generated in the methods of the present invention enables rapid assignment of a and b ion types to the peaks in the fragmentation mass spectra. For example, in Figure 3a, the peaks that form the a₁/b₁, a₂/b₂, a₃/b₃, a₅/b₅, a₆/b₆ and a₇/b₇ a/b doublets are visible, and are separated by the mass of a carbonyl group (28 Da). A similar number of a/b doublets is visible in Figure 3b. In addition, the m/z separation between adjacent pairs of a/b doublets corresponds to that of an amino acid, enabling an amino acid sequence to be assigned to the peptide in that manner.

Figure 4 shows the ESI-Q-TOF-MS/MS mass spectrum of GluFib B peptide (Figure 4a) without and (Figure 4b) with b and y ion series marked, and illustrates that the fragmentation mass spectra generated in the methods of the present invention may contain peaks corresponding to each of a, b and y daughter ions. In Figure 4, a complete y ion series is visible (annotated in Figure 4b) in addition to the a and b ion series. Figure 5 shows the daughter ion types generated by backbone fragmentation of the GluFib B peptide whose mass spectrum is shown in Figure 4, alongside the daughter ion types normally detected in the mass spectrum of the same peptide when unlabelled. As illustrated by Figures 4 and 5, it is only when fragmentation mass spectrometry is performed using the derivatised GluFib B peptide that peaks corresponding to each of a, b and y daughter ions are seen in the fragmentation mass spectrum.

### Example 4: Identification of proline residues by fragmentation mass spectrometry

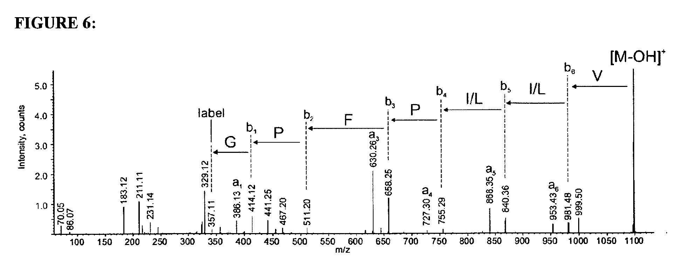

Figure 6 shows the MALDI-Q-TOF-CID-MS/MS mass spectrum of NH₂-GPFPIIV-COOH (SEQ ID NO:4), a proline-containing peptide. As illustrated by that Figure, the presence of peaks corresponding to both a and b daughter ions in the mass spectra generated in the methods of the present invention is not affected by the presence of a proline residue in a derivatised peptide. Indeed, the peptide whose mass spectrum is shown in Figure 6 contains two proline residues and yet it is still possible for an unambiguous amino acid sequence to be assigned to the peptide on the basis of the fragmentation mass spectrum.

It will be understood that the invention is described above by way of example only and modifications may be made whilst remaining within the scope and spirit of the invention.

## Claims

1. A method for analysing a peptide by mass spectrometry, comprising the step of obtaining a fragmentation mass spectrum for the peptide, wherein the fragmentation mass spectrum contains peaks corresponding to a and b daughter ions formed by cleavage between the same two amino acids but at different bonds.

2. A method according to claim 1, wherein the step of obtaining a fragmentation mass spectrum comprises the steps of
(a) reacting the peptide's N-terminus with a label to provide a derivatised peptide, wherein the label is an organic group that allows positive charge to be retained by a and b ions formed during fragmentation mass spectrometry of the derivatised peptide; and
(b) providing a fragmentation mass spectrum of the derivatised peptide.

3. A method according to claim 2, wherein step (b) is fragmentation mass spectrometry.

4. A method according to claim 3, wherein step (b) comprises the steps of:
(b1) performing mass spectrometry of the derivatised peptide to obtain a first mass spectrum;
(b2) identifying at least one parent ion in the first mass spectrum;
(b3) selecting at least one parent ion for further analysis by mass spectrometry; and
(b4) performing fragmentation mass spectrometry of the selected parent ion(s) to obtain a fragmentation mass spectrum, wherein the fragmentation mass spectrum contains peaks corresponding to both a and b ions derived from fragmentation of the derivatised peptide.

5. A method for determining an amino acid sequence of a peptide, comprising the steps of:
(a) obtaining a fragmentation mass spectrum for the peptide, wherein the fragmentation mass spectrum contains peaks corresponding to both a and b daughter ions;
(b) identifying peaks in the fragmentation mass spectrum of step (a) corresponding to a or b daughter ions; and
(c) assigning an amino acid sequence to the peptide on the basis of the m/z separation of consecutive peaks in the a or b ion series.

6. A method for analysing a peptide by mass spectrometry, comprising the step of obtaining a fragmentation mass spectrum for the peptide, wherein the fragmentation mass spectrum contains at least one a/b doublet.

7. A method according to claim 6, wherein the fragmentation mass spectrum contains a/b doublets for at least 50% of the peptide's amino acid residues.

8. A method for analysing a peptide by mass spectrometry, comprising the step of obtaining a fragmentation mass spectrum for the peptide, wherein the fragmentation mass spectrum contains peaks corresponding to each of a, b and y daughter ions.

9. A method according to claim 8, wherein the step of obtaining a fragmentation mass spectrum comprises the steps of:
(a) reacting the peptide's N-terminus with a label to provide a derivatised peptide, wherein the label is an organic group that allows positive charge to be retained by a and b ions formed during fragmentation mass spectrometry of the derivatised peptide; and
(b) providing a fragmentation mass spectrum of the derivatised peptide.

10. A method according to claim 9, wherein step (b) is fragmentation mass spectrometry.

11. A method according to claim 10, wherein step (b) comprises the steps of:
(b1) performing mass spectrometry of the derivatised peptide to obtain a first mass spectrum;
(b2) identifying at least one parent ion in the first mass spectrum;
(b3) selecting at least one parent ion for further analysis by mass spectrometry; and
(b4) performing fragmentation mass spectrometry of the selected parent ion(s) to obtain a fragmentation mass spectrum, wherein the fragmentation mass spectrum contains peaks corresponding to each of a, b and y ions derived from fragmentation of the peptide.

12. A method for determining an amino acid sequence of a peptide, comprising the steps of:
(a) obtaining a fragmentation mass spectrum for the peptide, wherein the fragmentation mass spectrum contains peaks corresponding to each of a, b and y daughter ions;
(b) identifying peaks in the fragmentation mass spectrum of step (a) corresponding to a, b or y daughter ions; and
(c) assigning an amino acid sequence to the peptide on the basis of the m/z separation of consecutive peaks in one or more of the a, b and y ion series.

13. A method according to claim 1 or claim 8, wherein the fragmentation mass spectrum contains a pair of consecutive peaks in the a, b or y ion series, with a m/z separation corresponding to a proline residue.

14. A method according to claim 13, wherein the step of obtaining a fragmentation mass spectrum comprises the steps of:
(a) reacting the peptide's N-terminus with a label to provide a derivatised peptide, wherein the peptide contains a proline residue and wherein the label stabilises daughter ions formed during fragmentation mass spectrometry and comprising a C-terminal proline residue and/or permits cleavage between the proline and the amino acid immediately C-terminal to it; and
(b) providing a fragmentation mass spectrum of the derivatised peptide.

15. A method according to claim 14, wherein step (b) is fragmentation mass spectrometry.

16. A method according to claim 15, wherein step (b) comprises the steps of:
(b1) performing mass spectrometry of the derivatised peptide to obtain a first mass spectrum;
(b2) identifying at least one parent ion in the first mass spectrum;
(b3) selecting at least one parent ion for further analysis by mass spectrometry; and
(b4) performing fragmentation mass spectrometry of the selected parent ion(s) to obtain a fragmentation mass spectrum, wherein the fragmentation mass spectrum contains a pair of consecutive peaks in the a, b or y ion series with a m/z separation corresponding to a proline residue.

17. A peptide with a N-terminal residue, **characterised in that** (a) a label that allows positive charge to be retained by a and b ions formed during fragmentation mass spectrometry of the peptide is attached to the N-terminal residue of the peptide and (b) the peptide provides a fragmentation mass spectrum that contains at least one a/b doublet.

18. A peptide according to claim 17, including a proline residue downstream of the N-terminal residue, **characterised in that** (a) a label that stabilises daughter ions formed during fragmentation mass spectrometry and comprising a C-terminal proline residue and/or permits cleavage between the proline and the amino acid immediately C-terminal to it is attached to the N-terminal residue of the peptide and (b) the peptide provides a fragmentation mass spectrum that contains a pair of consecutive peaks in the a, b or y ion series with a m/z separation corresponding to a proline residue.

19. A peptide according to claim 17 or claim 18, wherein the peptide is not a peptide consisting of the amino acid sequence NH₂-RHPEYAVSVLLR-COOH (SEQ ID NO:1) or NH₂-DDPHACYSTVFDK-COOH (SEQ ID NO:2).

20. The method according to any one of claims 3-4, 10-11 or 15-16, wherein the mass spectrometer is an ESI-Q-TOF mass spectrometer or a MALDI-Q-TOF mass spectrometer.

21. The method according to any one of claims 3-4, 10-11 or 15-16, wherein the fragmentation of the parent ion to produce daughter ions is induced by CID or PSD.

22. A method according to claim 5 or claim 12, further comprising the step of analysing a fragmentation mass spectrum to determine if it contains a peak pattern for an a or b ion in which a first peak and a second peak are separated by one average mass unit and in which the first peak is less abundant than the second peak and has a lower mass than the second peak.

23. A method according to claim 22, wherein the mass spectrum is a deisotoped spectrum and the first peak and second peak are monoisotopic mass peaks.

24. A method for identifying a polypeptide by mass spectrometry, comprising the steps of:
(a) obtaining a fragmentation mass spectrum of a peptide derived from a polypeptide, wherein the mass spectrum contains peaks corresponding to a and b ions derived from fragmentation of the peptide;
(b) identifying peaks in the fragmentation mass spectrum of step (a) corresponding to a or b daughter ions; and
(c) assigning an amino acid sequence to the peptide on the basis of the m/z separation of consecutive peaks in the a or b ion series; and
(d) searching a database using amino acid sequence information generated in step (c) to identify the polypeptide.

25. A method for identifying a polypeptide by mass spectrometry, comprising the steps of:
(a) obtaining a fragmentation mass spectrum of a peptide derived from a polypeptide, wherein the mass spectrum contains peaks corresponding to a, b and y ions derived from fragmentation of the peptide;
(b) identifying peaks in the fragmentation mass spectrum of step (a) corresponding to a, b or y daughter ions; and
(c) assigning an amino acid sequence to the peptide on the basis of the m/z separation of consecutive peaks in one or more of the a, b and y ion series; and
(d) searching a database using amino acid sequence information generated in step (c) to identify the polypeptide.

26. A method for identifying a polypeptide by mass spectrometry, comprising the steps of:
(a) obtaining a first mass spectrum of a mixture of peptides derived from a polypeptide;
(b) identifying monoisotopic masses of the peptides in the first mass spectrum;
(c) selecting one or more parent ions for further analysis by mass spectrometry;
(d) obtaining a fragmentation mass spectrum for the parent ion(s) selected in step (c), wherein the fragmentation mass spectrum contains peaks corresponding to a and b ions;
(d) identifying peaks in the fragmentation mass spectrum of step (c) corresponding to a or b ions;
(e) assigning an amino acid sequence to the selected parent ion(s) on the basis of the m/z separation of consecutive peaks in the a or b ion series; and
(f) searching a database using information generated in steps (b) and (e) to identify the polypeptide.

27. A method for identifying a polypeptide by mass spectrometry, comprising the steps of:
(a) obtaining a first mass spectrum of a mixture of peptides derived from a polypeptide;
(b) identifying monoisotopic masses of the peptides in the first mass spectrum;
(c) selecting one or more parent ions for further analysis by mass spectrometry;
(d) obtaining a fragmentation mass spectrum for the parent ion(s) selected in step (c), wherein the fragmentation mass spectrum contains peaks corresponding to a, b and y ions;
(d) identifying peaks in the fragmentation mass spectrum of step (c) corresponding to a, b or y ions;
(e) assigning an amino acid sequence to the selected parent ion(s) on the basis of the m/z separation of consecutive peaks in one or more of the a, b and y ion series; and
(f) searching a database using information generated in steps (b) and (e) to identify the polypeptide.
